# EUROPEAN PATENT APPLICATION

(11) **EP 4 830 774 A1**
(43) Date of publication of application: **28.10.2026**
(21) Application number: 26172318.3
(22) Date of filing: 14.04.2026
(51) Int. Cl.: A61N 5/10

(54) **RADIATION TREATMENT PLAN OPTIMIZATION METHOD AND APPARATUS**

(30) Priority: 22.04.2025 US 202519186281
(71) Applicant: Siemens Healthineers International AG, 6312 Steinhausen (CH)
(72) Inventor: PELTOLA, Jarkko, 00100 Helsinki (FI); TALLINEN, Tuomas, 00100 Helsinki (FI)
(74) Representative: Mathisen & Macara LLP

(57) **Abstract**

A control circuit (101) accesses (201) patient geometry information that includes at least one target volume along with radiation treatment apparatus geometry information for a particular radiation treatment apparatus and radiation direction field geometry setup parameters that include at least one field geometry setup with at least one gantry angle, one couch angle, and one isocenter position. The control circuit (101) can then automatically determine (204) multi-leaf collimator (MLC) plane field geometry setup parameters for at least one radiation direction field geometry setup using a single automated multiple-field setup per radiation direction field followed by optimizing the radiation treatment plan as a function of the MLC plane field geometry setup parameters to provide (207) an optimized radiation treatment plan.

## Description

### TECHNICAL FIELD

These teachings relate generally to treating a patient's target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Radiation treatment planning often includes initially setting up radiation fields so that the fields will allow maximum target structure dose coverage with minimal negative features, such as abnormal tissue dosing and/or undue treatment time, while also observing physical limitations of the radiation treatment machine itself. Setting up these radiation fields is, at least to some extent, a geometrical problem.

### SUMMARY OF INVENTION

In accordance with a first aspect of the invention, there is provided a method of optimizing a radiation treatment plan, as defined by claim 1.

In accordance with a second aspect of the invention, there is provided an apparatus for optimizing a radiation treatment plan, as defined by claim 8.

Optional features are defined by the dependent claims.

### BRIEF DESCRIPTION OF DRAWINGS

Various needs are at least partially met through provision of the radiation treatment plan optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic representation as configured in accordance with at least some prior art approaches; and
FIG. 4 comprises a schematic representation as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments can provide for optimizing a radiation treatment plan for a particular patient using a particular radiation treatment apparatus having a radiation source. By one approach, a control circuit accesses patient geometry information that includes at least one target volume along with radiation treatment apparatus geometry information for the particular radiation treatment apparatus and radiation direction field geometry setup parameters that include at least one field geometry setup with at least one gantry angle, one couch angle, and one isocenter position. The control circuit can then automatically determine multi-leaf collimator (MLC) plane field geometry setup parameters for at least one radiation direction field geometry setup using a single automated multiple-field setup per radiation direction field followed by optimizing the radiation treatment plan as a function of the MLC plane field geometry setup parameters to provide an optimized radiation treatment plan. By one approach, the foregoing "field" can comprise an arc field.

By one approach, the aforementioned single automated multiple-field setup per radiation direction field can comprise a single automated two-field setup per isocenter.

By one approach, automatically determining MLC plane field geometry setup parameters for at least one radiation direction field geometry setup using a single automated multiple-field setup per radiation direction field can comprise using a single automated multiple-field setup for each isocenter and arc field geometry setup comprising at least one start gantry angle and one end gantry angle. For example, optionally, a radiation direction field may comprise an isocenter, start gantry angle and/or one end gantry angle.

By one approach, automatically determining at least one radiation direction field geometry setup using a single automated multiple-field setup per radiation direction field can comprise, at least in part, generating pairs of arc fields that overlap in angle space while having field opening limiting apertures that are set so that the target volume, for example and optionally the whole target volume, is encompassed.

By one approach, automatically determining MLC plane field geometry setup parameters for at least one radiation direction field geometry setup using a single automated multiple-field setup per radiation direction field can comprise, at least in part, dividing the multiple-field setups between treatment fractions such that only one field pair, with one corresponding collimator angle setup, at a time is used to produce a dose result in each treatment fraction. In such a case, if desired, optimizing the radiation treatment plan as a function of the MLC plane field geometry setup parameters can comprise allowing use of all geometrical degrees of freedom of the particular radiation treatment apparatus including multi-leaf collimator leaf angles with respect to structure borders.

By one optional approach, these teachings will accommodate accessing a user-input value for a collimator angle. In that case, optimizing the radiation treatment plan as a function of the MLC plane field geometry setup parameters can comprise optimizing the radiation treatment plan as a function of the MLC plane field geometry setup parameters and the user-input value for the collimator angle.

In lieu of the foregoing, these teachings will also accommodate automatically determining at least one collimator angle to provide at least one automatically determined collimator angle. In that case, optimizing the radiation treatment plan as a function of the MLC plane field geometry setup parameters can comprise optimizing the radiation treatment plan as a function of the MLC plane field geometry setup parameters and the at least one automatically determined collimator angle.

It will be noted, however, that these teachings will accommodate accessing no user-input values for field opening settings for the MLC plane itself. For example, the present method may not access user-input values for field opening settings for the MLC plane.

These teachings will also optionally accommodate administering therapeutic radiation to the particular patient as a function of the optimized radiation treatment plan.

By one approach, these teachings can comprise a non-transitory computerreadable medium having a computer program that itself comprises instructions that, when the computer program is executed by a computer, causes the computer to carry out accessing patient geometry information that includes at least one target volume along with radiation treatment apparatus geometry information for the particular radiation treatment apparatus and radiation direction field geometry setup parameters that include at least one field geometry setup with at least one gantry angle, one couch angle, and one isocenter position, followed by automatically determining multi-leaf collimator (MLC) plane field geometry setup parameters for at least one radiation direction field geometry setup using a single automated multiple-field setup per radiation direction field and then optimizing the radiation treatment plan as a function of the MLC plane field geometry setup parameters to provide an optimized radiation treatment plan.

So configured, these teachings can provide, for example, for automatic radiation treatment plan setup using double fields and fractionation. By one approach, these teachings can be applied with complicated treatments where, for example, gantry rotation pauses during a treatment field. These teachings will also accommodate automatically dividing field pairs amongst different collimator pairs which are then divided for use in different treatment fractions.

Before describing these teachings in more detail, it may be helpful to understand that, when setting up treatment fields, it typically will not be clear to the clinician how different setups, especially in cases where there are multiple fields that each only partially cover sections of a large target structure, will correlate with achievable plan quality. As a result, the planning process may include iterating through the entirety of the planning process to find a satisfactory setup. That approach, however, typically relies on essentially guessing the field setup parameters, launching a dose optimization algorithm, and checking the results of a three-dimensional dose against the relevant structures. This trial and error process requires time and resources and itself can lead to variations in achieved plan qualities over different patients and planners.

Another point of concern is lack of a trusted, templatized method for setting up fields that would work well over all different patient geometries. In addition, typically existing templatized methods have many free parameter fields that the clinician needs to select, which essentially obviates the primary benefit of employing a template.

It can also be noted that a typical planning approach will use the same geometry throughout the whole treatment. As a result, and by way of example, while a treatment can constitute thirty treatment fractions, each treatment fraction will use the same field geometry. The applicant has determined that such an approach is not always optimal.

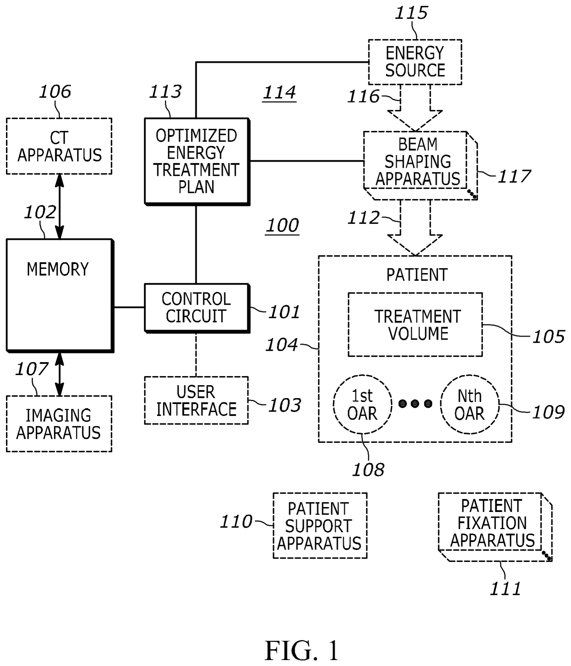

Various benefits of these teachings in comparison to the foregoing may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as the aforementioned patient geometry information that includes at least one target volume for a particular patient, radiation treatment apparatus geometry information for a particular radiation treatment apparatus, and radiation direction field geometry setup parameters that include at least one field geometry setup with at least one gantry angle, one couch angle, and one isocenter position as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patientrelated imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

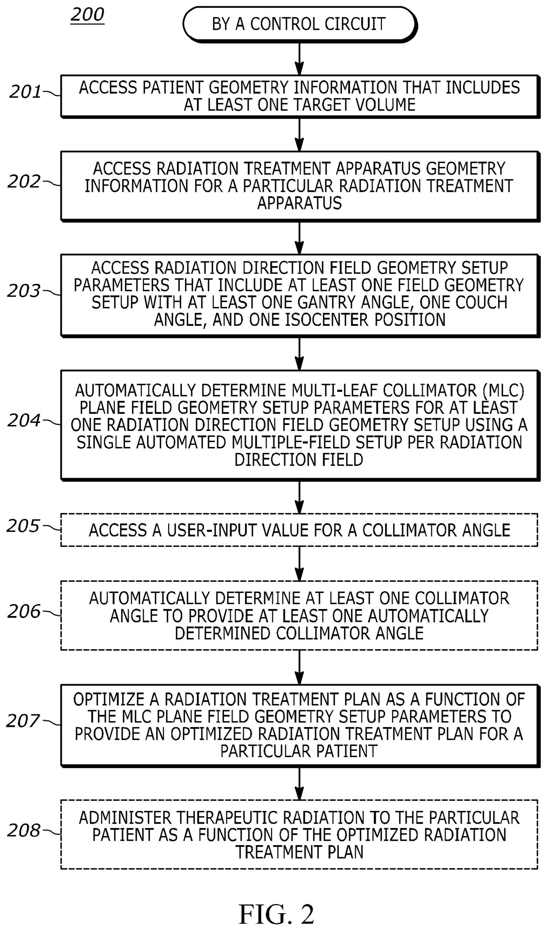

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan. For example, the method may automatically generate an optimized radiation treatment plan.

At block 201, the control circuit 101 accesses patient geometry information that includes at least one target volume. That information (like other information described herein) may be accessed, for example, by communicating with the aforementioned memory 102. In addition to a target volume, the patient geometry information may include other information regarding such things as one or more untargeted organs-at-risk. The accessed patient geometry information may include image information such as one or more computed tomography images.

At block 202, the control circuit 101 accesses radiation treatment apparatus geometry information for a particular radiation treatment apparatus (in particular, the radiation treatment apparatus that is planned for use when administering the radiation treatment to the particular patient). Such geometry information generally comprises information pertaining to the spatial arrangement and/or physical characteristics of the equipment that comprises the radiation treatment apparatus and that influences the delivery of radiation to the target volume.

And at block 203, the control circuit 101 accesses radiation direction field geometry setup parameters that include at least one field geometry setup with at least one gantry angle, at least one couch angle, and at least one isocenter position. The isocenter position refers to the isocenter, which is a defined (e.g. precisely defined) point in space around which the radiation delivery system is designed to rotate. The isocenter serves as the focal point for the beams of radiation that are delivered from multiple angles to converge on the target volume. In many application settings, the patient is positioned so that the target volume aligns with the machine's isocenter.

At block 204, the control circuit 101 then automatically determines multi-leaf collimator plane field geometry setup parameters for at least one radiation direction field geometry setup using a single automated multiple-field setup per radiation direction field. By one approach, if desired, that single automated multiple-field setup per radiation direction field comprises a single automated two-field setup per isocenter, but these teachings will accommodate N-field setups per isocenter (where "N" is an integer equal to or greater than 2).

By one approach, the aforementioned automatic determination of multi-leaf collimator plane field geometry setup parameters for at least one radiation direction field geometry setup using a single automated multiple-field setup per radiation direction field can comprise using a single automated multiple-field setup for each isocenter and arc field geometry setup comprising at least one start gantry angle and one end gantry angle.

By one approach, the aforementioned automatic determination of at least one radiation direction field geometry setup using a single automated multiple-field setup per radiation direction field can comprise, at least in part, generating pairs of arc fields that overlap in angle space while having field opening limiting apertures that are set so that the target volume is encompassed. By one approach, the target volume is fully encompassed. By another approach, the target volume is at least substantially encompassed, where "substantially encompassed" can refer to 80% encompassed, 85% encompassed, 90% encompassed, or 95% encompassed as desired, or any value within a range of, for example, 75% to 100% encompassed as may be appropriate to a given application setting.

By one approach, the aforementioned automatic determination of multi-leaf collimator plane field geometry setup parameters for at least one radiation direction field geometry setup using a single automated multiple-field setup per radiation direction field can comprise, at least in part, dividing the multiple-field setups between treatment fractions such that only one field pair, with one corresponding collimator angle setup, at a time is used to produce a dose result in each treatment fraction.

At optional block 205, the control circuit 101 can access a user-input value for at least one collimator angle that can be used during the optimization process. A user may input such a value, for example, by utilizing the above-described user interface 103. The collimator angle is the angle at which the multi-leaf collimator rotates around the beam's axis. Changing the collimator angle can adjust the orientation of the multi-leaf collimator leaves with respect to the target volume and the surrounding tissues.

In lieu of the foregoing optional use of a user-input value for at least one collimator angle, or in combination therewith, at optional block 206 the control circuit 101 can automatically determine at least one collimator angle to provide at least one automatically determined collimator angle that can be used during the optimization process. These teachings will accommodate various approaches to making this automatic determination. Examples include, but are not limited to, calculating the angle, looking up the angle in a table of angle values, prompting a machine learning model for the angle value, and so forth.

At block 207, the control circuit 101 optimizes a radiation treatment plan for the particular patient using the aforementioned radiation treatment apparatus as a function of the aforementioned multi-leaf collimator plane field geometry setup parameters to thereby provide an optimized radiation treatment plan. By one approach, this optimization of the radiation treatment plan as a function of the multi-leaf collimator plane field geometry setup parameters can comprise allowing use of all geometrical degrees of freedom of the particular radiation treatment apparatus including multi-leaf collimator leaf angles with respect to structure borders.

Those skilled in the art will note that this process 200 provides for accessing no user-input values for field opening settings for the multi-leaf collimator plane. The absence of such a step runs contrary to typical prior art practice in these regards.

If desired, and as shown at optional block 208, this process 200 will accommodate administering therapeutic radiation to the particular patient as a function of the aforementioned optimized radiation treatment plan.

Further details that comport with these teachings will now be presented. It will be understood that the specific details of these examples are intended to serve an illustrative purpose and are not intended to suggest any particular limitations with respect to these teachings.

By one approach, these teachings can provide for solving some or all of the arc field geometry setup problems in some or all treatment sites using a single automatized two-field setup per iso-center. This approach can almost completely offload the burden of field geometry setup from the planner. For example, the field geometry setup may be performed automatically. This approach can also reduce variations in achieved plan quality and yield a better plan than approaches that require significant manually-based setups.

By one approach, these teachings can include one free parameter, a collimator angle, for the user to set. The effect on plan quality of that choice is limited, particularly as compared to having multiple manually-set collimator angles. In application settings where the radiation treatment machine and planning system support optimized, dynamically changing collimator angles, then even this free parameter can be removed. For example, the radiation treatment machine and the planning system may be configured to optimize dynamically changing collimator angles, optionally without a user-defined collimator angle. In application settings lacking dynamic collimator angles, the present teachings can be extended to use a two-field setup so that the collimator angle is nevertheless varied between treatment fractions.

An illustrative example of an automatic two field setup will now be provided.

This example presumes a not untypical head and neck treatment site, where the target size is greater than 20 cm and accordingly exceeds the maximum multi-leaf collimator leaf span of 15 cm as applies to many treatment machines. Leaf span refers to the maximum distance that a multi-leaf collimator leaf can extend and freely modulate dose within one field. Thus, the leaf span width is typically used as the maximum field size in the X-direction when setting up the fields.

This example presumes that the maximum field size limit is given as a rectangular region using (x1,x2,y1,y2) positions in the multi-leaf collimator plane. This rectangular region can be referred to as a "field aperture." In arc fields, the target projection in the multi-leaf collimator plane can change with each gantry angle and the projection will be outside of the set field aperture in some angles and inside in some angles.

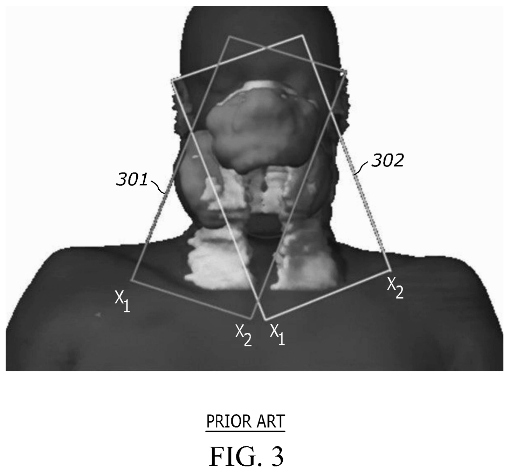

To accommodate the missing parts of the target projection, a prior art user typically sets up another arc field that has a different collimator angle and field aperture so that the combination of the two arc fields, still limited by the 15cm field X-size, will eventually cover all target projections in all gantry angles. There are typically multiple combinations for the set parameters (collimator angles, field apertures) that will achieve the necessary coverage but their effect on the achievable plan quality is unknown. FIG. 3 presents one illustrative example in these regards.

In particular, FIG. 3 illustrates a prior art approach that employs a manual way to set up arc fields for the aforementioned head-neck case, where the target projection exceeds the 15 cm range when viewed from any gantry angle. The two rectangles 301 and 302 represent the X-range of maximum leaf span (x1 .. x2 = 15cm), which is the maximum range where the multi-leaf collimator leaves can fully modulate. Here, the user must figure out the combination of collimator angles for both fields (here, the x1 and x2 positions for both fields).

In contrast to the foregoing, the present teachings, after setting the iso-center, only require the user to set the angle setup for the arc field (such as a collimator angle and start/stop angles for the arc). Nothing is required from the user as regards the field aperture. The automatic tool can then create pairs of arc fields that overlap in angle space but where the field apertures are set so that the whole target structure can always be covered. This setup can be done regardless of how large the target volume may be.

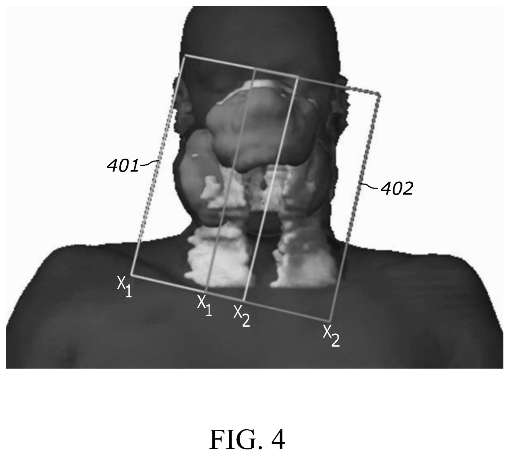

FIG. 4 presents an illustrative example in these regards that presumes the same head-neck case presented in FIG. 3. In this example, each field has the X-range 401 and 402 set according to the maximum leaf span but here, the fields can be combined so that the combination covers the entire target volume from all gantry angles. So configured, the user has only one parameter to set: the common collimator angle. By one approach, the collimator angle can have a nonzero default value, for example 30 or 45 degrees, which can be suitable for at least some and perhaps most patient cases. (In cases where a dynamic collimator rotation can be used, even that one manual choice can be omitted.)

Such an approach makes the arc field setup an easy and standardized process. This approach can also reduce achieved plan quality variance between different planners/manual setups.

By one approach, the foregoing automation can comprise projecting the selected target volumes onto the multi-leaf collimator plane in each gantry angle of the field, combining the projection two-dimensional maps to form a single cumulative projection map, and then setting the rectangular field apertures so that the first field's leftmost X1 edge covers the outermost left side of the target projection map and setting the X2 edge to be at maximum leaf span distance from that. The other field would be generated symmetrically but leaning to the outermost right side of the target. In cases where the target volume is less than the maximum span, both fields will fully overlap if not otherwise advised. The arc rotation direction for each field in the plan can vary between clock-wise and counter-clock-wise rotation if desired to reduce the overall treatment time.

An illustrative example of an automatic fraction-based setup will now be provided.

By another approach, these teachings will accommodate eliminating the last remaining free parameter, the collimator angle. The collimator angle space should ordinarily be covered from at least a few angles if a universally-working setup is to be created. This feature could be accommodated simply by creating multiple field-pairs, each with a different collimator angle. Unfortunately, if all of those field-pairs were used for optimizing the total dose, the treatment time would be multiplied accordingly.

Instead, these teachings will accommodate dividing the field setup between treatment fractions so that only one double field pair, with one collimator angle, at a time is used to produce the dose in each treatment fraction. How the fields are distributed per fraction can vary according to treatment and user needs.

As one illustrative example in the foregoing regards, if the plan is to treat with 5 times 10 Gy fraction dose, one can use one double arc setup with collimator angle 0 degrees for 1 x 10Gy, then another pair with angle 22.5deg for 1 x 10Gy, another with 45 deg for 1 x 10Gy, one with 67.5deg and one with 90deg for 1 x 10Gy. The end result is that both total dose and treatment time remains the same, but the radiation treatment plan has more degrees of freedom to permit adjustments to account for patient geometry.

One benefit of a fraction-based setup is that all of the geometrical degrees of freedom can be used by the optimizer, including how multi-leaf collimator leaves are angled with respect to structure borders, and the user will not need to set up any parameters that carry with them unknown effects to achievable plan quality.

Further aspects of these teachings are provided by the subject matter of the following clauses (where it will be understood that any of these clauses can be combined with any one of more of the other clauses as desired).

Clause 1. A method of optimizing a radiation treatment plan for a particular patient using a particular radiation treatment apparatus having a radiation source, the method comprising: by a control circuit: accessing patient geometry information that includes at least one target volume; accessing radiation treatment apparatus geometry information for the particular radiation treatment apparatus; accessing radiation direction field geometry setup parameters that include at least one field geometry setup with at least one gantry angle, one couch angle, and one isocenter position; automatically determining multi-leaf collimator (MLC) plane field geometry setup parameters for at least one radiation direction field geometry setup using a single automated multiple-field setup per radiation direction field; optimizing the radiation treatment plan as a function of the MLC plane field geometry setup parameters to provide an optimized radiation treatment plan.

Clause 2. The method of clause 1 wherein automatically determining MLC plane field geometry setup parameters for at least one radiation direction field geometry setup using a single automated multiple-field setup per radiation direction field comprises using a single automated multiple-field setup for each isocenter and arc field geometry setup comprising at least one start gantry angle and one end gantry angle.

Clause 3. The method of clause 1 or clause 2 further comprising: accessing a user-input value for a collimator angle; and wherein optimizing the radiation treatment plan as a function of the MLC plane field geometry setup parameters comprises optimizing the radiation treatment plan as a function of the MLC plane field geometry setup parameters and the user-input value for the collimator angle.

Clause 4. The method of any preceding clause further comprising: automatically determining at least one collimator angle to provide at least one automatically determined collimator angle; and wherein optimizing the radiation treatment plan as a function of the MLC plane field geometry setup parameters comprises optimizing the radiation treatment plan as a function of the MLC plane field geometry setup parameters and the at least one automatically determined collimator angle.

Clause 5. The method of any preceding clause further comprising: accessing no user-input values for field opening settings for the MLC plane.

Clause 6. The method of any preceding clause wherein automatically determining at least one radiation direction field geometry setup using a single automated multiple-field setup per radiation direction field comprises, at least in part, generating pairs of arc fields that overlap in angle space while having field opening limiting apertures that are set so that the target volume is encompassed.

Clause 7. The method of any preceding clause wherein the single automated multiple-field setup per radiation direction field comprises a single automated two-field setup per isocenter.

Clause 8. The method of any preceding clause wherein automatically determining MLC plane field geometry setup parameters for at least one radiation direction field geometry setup using a single automated multiple-field setup per radiation direction field comprises, at least in part, dividing the multiple-field setups between treatment fractions such that only one field pair, with one corresponding collimator angle setup, at a time is used to produce a dose result in each treatment fraction.

Clause 9. The method of clause 8 wherein optimizing the radiation treatment plan as a function of the MLC plane field geometry setup parameters comprises allowing use of all geometrical degrees of freedom of the particular radiation treatment apparatus including multi-leaf collimator leaf angles with respect to structure borders.

Optional Clause 10. The method of any preceding clause further comprising: administering therapeutic radiation to the particular patient as a function of the optimized radiation treatment plan.

Clause 11. An apparatus for optimizing a radiation treatment plan for a particular patient using a particular radiation treatment apparatus having a radiation source, the apparatus comprising: a control circuit configured to: access patient geometry information that includes at least one target volume; access radiation treatment apparatus geometry information for the particular radiation treatment apparatus; access radiation direction field geometry setup parameters that include at least one field geometry setup with at least one gantry angle, one couch angle and one isocenter position; automatically determine multi-leaf collimator (MLC) plane field geometry setup parameters for at least one radiation direction field geometry setup using a single automated multiple-field setup per radiation direction field; optimize the radiation treatment plan as a function of the MLC plane field geometry setup parameters to provide an optimized radiation treatment plan.

Clause 12. The apparatus of clause 11 wherein the control circuit is configured to automatically determine MLC plane field geometry setup parameters for at least one radiation direction field geometry setup using a single automated multiple-field setup per radiation direction field by using a single automated multiple-field setup for each isocenter and arc field geometry setup comprising at least one start gantry angle and one end gantry angle.

Clause 13. The apparatus of clause 11 or clause 12 wherein the control circuit is further configured to: access a user-input value for a collimator angle; and wherein the control circuit is configured to optimize the radiation treatment plan as a function of the MLC plane field geometry setup parameters by optimizing the radiation treatment plan as a function of the MLC plane field geometry setup parameters and the user-input value for the collimator angle.

Clause 14. The apparatus of any of clause 11 to clause 13 wherein the control circuit is further configured to: automatically determine at least one collimator angle to provide at least one automatically determined collimator angle; and wherein the control circuit is configured to optimize the radiation treatment plan as a function of the MLC plane field geometry setup parameters by optimizing the radiation treatment plan as a function of the MLC plane field geometry setup parameters and the at least one automatically determined collimator angle.

Clause 15. The apparatus of any of clause 11 to clause 14 wherein the control circuit is further configured to: access no user-input values for field opening settings for the MLC plane.

Clause 16. The apparatus of any of clause 11 to clause 15 wherein the control circuit is configured to automatically determine at least one radiation direction field geometry setup using a single automated multiple-field setup per radiation direction field by, at least in part, generating pairs of arc fields that overlap in angle space while having field opening limiting apertures that are set so that the target volume is encompassed.

Clause 17. The apparatus of any of clause 11 to clause 16 wherein the single automated multiple-field setup per radiation direction field comprises a single automated two-field setup per isocenter.

Clause 18. The apparatus of any of clause 11 to clause 17 wherein the control circuit is configured to automatically determine MLC plane field geometry setup parameters for at least one radiation direction field geometry setup using a single automated multiple-field setup per radiation direction field by, at least in part, dividing the multiple-field setups between treatment fractions such that only one field pair, with one corresponding collimator angle setup, at a time is used to produce a dose result in each treatment fraction.

Clause 19. The apparatus of clause 18 wherein the control circuit is configured to optimize the radiation treatment plan as a function of the MLC plane field geometry setup parameters by allowing use of all geometrical degrees of freedom of the particular radiation treatment apparatus including multi-leaf collimator leaf angles with respect to structure borders.

Clause 20. The apparatus of any of clause 11 to clause 19 wherein the control circuit is further configured to: administer therapeutic radiation to the particular patient as a function of the optimized radiation treatment plan.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method of optimizing a radiation treatment plan for a particular patient using a particular radiation treatment apparatus having a radiation source, the method comprising:
by a control circuit:
accessing patient geometry information that includes at least one target volume;
accessing radiation treatment apparatus geometry information for the particular radiation treatment apparatus;
accessing radiation direction field geometry setup parameters that include at least one field geometry setup with at least one gantry angle, one couch angle, and one isocenter position;
automatically determining multi-leaf collimator (MLC) plane field geometry setup parameters for at least one radiation direction field geometry setup using a single automated multiple-field setup per radiation direction field;
optimizing the radiation treatment plan as a function of the MLC plane field geometry setup parameters to provide an optimized radiation treatment plan.

2. The method of claim 1 wherein automatically determining MLC plane field geometry setup parameters for at least one radiation direction field geometry setup using a single automated multiple-field setup per radiation direction field comprises using a single automated multiple-field setup for each isocenter and arc field geometry setup comprising at least one start gantry angle and one end gantry angle.

3. The method of claim 1 or claim 2 further comprising:
accessing a user-input value for a collimator angle;
and wherein optimizing the radiation treatment plan as a function of the MLC plane field geometry setup parameters comprises optimizing the radiation treatment plan as a function of the MLC plane field geometry setup parameters and the user-input value for the collimator angle.

4. The method of any preceding claim further comprising:
automatically determining at least one collimator angle to provide at least one automatically determined collimator angle;
and wherein optimizing the radiation treatment plan as a function of the MLC plane field geometry setup parameters comprises optimizing the radiation treatment plan as a function of the MLC plane field geometry setup parameters and the at least one automatically determined collimator angle;
and/or:
the method further comprising:
accessing no user-input values for field opening settings for the MLC plane.

5. The method of any preceding claim wherein automatically determining at least one radiation direction field geometry setup using a single automated multiple-field setup per radiation direction field comprises, at least in part, generating pairs of arc fields that overlap in angle space while having field opening limiting apertures that are set so that the target volume is encompassed;
and/or:
wherein the single automated multiple-field setup per radiation direction field comprises a single automated two-field setup per isocenter.

6. The method of any preceding claim wherein automatically determining MLC plane field geometry setup parameters for at least one radiation direction field geometry setup using a single automated multiple-field setup per radiation direction field comprises, at least in part, dividing the multiple-field setups between treatment fractions such that only one field pair, with one corresponding collimator angle setup, at a time is used to produce a dose result in each treatment fraction.

7. The method of claim 6 wherein optimizing the radiation treatment plan as a function of the MLC plane field geometry setup parameters comprises allowing use of all geometrical degrees of freedom of the particular radiation treatment apparatus including multi-leaf collimator leaf angles with respect to structure borders.

8. An apparatus for optimizing a radiation treatment plan for a particular patient using a particular radiation treatment apparatus having a radiation source, the apparatus comprising:
a control circuit configured to:
access patient geometry information that includes at least one target volume;
access radiation treatment apparatus geometry information for the particular radiation treatment apparatus;
access radiation direction field geometry setup parameters that include at least one field geometry setup with at least one gantry angle, one couch angle and one isocenter position;
automatically determine multi-leaf collimator (MLC) plane field geometry setup parameters for at least one radiation direction field geometry setup using a single automated multiple-field setup per radiation direction field;
optimize the radiation treatment plan as a function of the MLC plane field geometry setup parameters to provide an optimized radiation treatment plan.

9. The apparatus of claim 8 wherein the control circuit is configured to automatically determine MLC plane field geometry setup parameters for at least one radiation direction field geometry setup using a single automated multiple-field setup per radiation direction field by using a single automated multiple-field setup for each isocenter and arc field geometry setup comprising at least one start gantry angle and one end gantry angle.

10. The apparatus of claim 8 or claim 9 wherein the control circuit is further configured to:
access a user-input value for a collimator angle;
and wherein the control circuit is configured to optimize the radiation treatment plan as a function of the MLC plane field geometry setup parameters by optimizing the radiation treatment plan as a function of the MLC plane field geometry setup parameters and the user-input value for the collimator angle.

11. The apparatus of any of claim 8 to claim 10 wherein the control circuit is further configured to:
automatically determine at least one collimator angle to provide at least one automatically determined collimator angle;
and wherein the control circuit is configured to optimize the radiation treatment plan as a function of the MLC plane field geometry setup parameters by optimizing the radiation treatment plan as a function of the MLC plane field geometry setup parameters and the at least one automatically determined collimator angle;
and/or:
wherein the control circuit is further configured to:
access no user-input values for field opening settings for the MLC plane.

12. The apparatus of any of claim 8 to claim 11 wherein the control circuit is configured to automatically determine at least one radiation direction field geometry setup using a single automated multiple-field setup per radiation direction field by, at least in part, generating pairs of arc fields that overlap in angle space while having field opening limiting apertures that are set so that the target volume is encompassed;
and/or:
wherein the single automated multiple-field setup per radiation direction field comprises a single automated two-field setup per isocenter.

13. The apparatus of any of claim 8 to claim 12 wherein the control circuit is configured to automatically determine MLC plane field geometry setup parameters for at least one radiation direction field geometry setup using a single automated multiple-field setup per radiation direction field by, at least in part, dividing the multiple-field setups between treatment fractions such that only one field pair, with one corresponding collimator angle setup, at a time is used to produce a dose result in each treatment fraction.

14. The apparatus of claim 13 wherein the control circuit is configured to optimize the radiation treatment plan as a function of the MLC plane field geometry setup parameters by allowing use of all geometrical degrees of freedom of the particular radiation treatment apparatus including multi-leaf collimator leaf angles with respect to structure borders.

15. The apparatus of any of claim 8 to claim 14 wherein the control circuit is further configured to:
administer therapeutic radiation to the particular patient as a function of the optimized radiation treatment plan.
